# EUROPEAN PATENT SPECIFICATION

(11) **EP 1 530 643 B1**
(45) Date of publication and mention of the grant of the patent: **04.05.2011**
(21) Application number: 03788682.7
(22) Date of filing: 14.08.2003
(51) Int. Cl.: C12P 21/06

(54) **CHIMERIC PROTEIN**
CHIMÄRISCHES PROTEIN
PROTEINE CHIMERE

(30) Priority: 15.08.2002 US 403839 P
(43) Date of publication of application: 18.05.2005
(73) Proprietor: Acorda Therapeutics, Inc., Hawthorne, NY 10532 (US)
(72) Inventor: GRUSKIN, Elliott, A., Malvern, PA 19355-9001 (US); GARGI, Roy, Danbury, CT 06811 (US); CHOJNICKI, Eric, Theodore, White Plains, NY 10606 (US)
(74) Representative: D'Arcy, Julia
(86) International application number: PCT/US2003/026214
(87) International publication number: WO 2004/017044

(56) References cited:
- EP-A2- 0 704 532
- WO-A1-95/13091
- ZUO J ET AL: "Regeneration of Axons after Nerve Transection Repair is Enhanced by Degradation of Chondroitin Sulfate Proteoglycan" EXPERIMENTAL NEUROLOGY, ACADEMIC PRESS, NEW YORK, NY, US, vol. 176, July 2002 (2002-07), pages 221-228, XP003001915 ISSN: 0014-4886
- MOON L.D.F. ET AL: 'Regeneration of CNS axons back to their target following treatment of adult rat brain with chondroitinase ABC' NAT. NEUROSCI. vol. 4, no. 5, May 2001, pages 465 - 466, XP008018581
- YICK L.W. ET AL: 'Chondroitinase ABC promotes axonal regeneration of Clarke's neurons after spinal cord injury' NEUROREPORT vol. 11, no. 5, 07 April 2000, pages 1063 - 1067, XP002978661
- YANG EV ET AL: 'Expression of Mmp-9 and related matrix metalloproteinase genes during axolotl limb regeneration' DEVELOPMENTAL DYNAMICS vol. 216, 1999, pages 2 - 9, XP002978625
- YANG EV ET AL: 'Developmental regulation of a matrix metalloproteinase during regeneration of axolotl appendages' DEV. BIOL. vol. 166, 1994, pages 696 - 703, XP002978474
- TONA ET AL: 'Effect of hyaluronidase on brain extracellular matrix in vivo and optic nerve regeneration' J. NEUROSCI. RES. vol. 36, October 1993, pages 191 - 199, XP002978696
- CHEN YS ET AL: 'Peripheral nerve regeneration using silicone rubber chambers filled with collagen, laminin and fibronectin' BIOMATERIALS vol. 21, 2000, pages 1541 - 1547, XP004204628
- MATSUMOTO K. ET AL: 'Peripheral nerve regeneration across an 80-mm gap bridged by a polyglycolic acid (PGA)-collagen tube filled with laminin-coated collagen fibers: a histological and electrophysiological evaluation of regenerated nerves' BRAIN RES. vol. 868, June 2000, pages 315 - 328, XP002978475
- STERNE GD ET AL: 'Neurotrophin-3 delivered locally via fibronectin mats enhances peripheral nerve regeneration' EUROPEAN JOURNAL OF NEUROSCIENCE vol. 9, no. 7, July 1997, pages 1388 - 1396, XP002978623
- TRIGG DJ ET AL: 'Peripheral nerve regeneration: comparison of laminin and acidic fibroblast growth factor' AM. J. OTOLARYNGOL. vol. 19, no. 1, January 1998 - February 1998, pages 29 - 32, XP002978476
- IWAI Y ET AL: 'Axon patterning requires DN-cadherin, a novel neuronal adhesion receptor, in the Drosophila embryonic CNS' NEURON vol. 19, July 1997, pages 77 - 89, XP002978477

## Description

### BACKGROUND

### Technical Field

The present disclosure relates to a chimeric protein containing at least two functional domains. The protein is encoded by a recombinant gene that possesses at least one domain coding a first polypeptide possessing matrix modification activity by removal of components of the extracellular matrix which inhibit neuronal growth and development and which is selected from matrix digesting enzymes and at least one other domain coding a second polypeptide possessing neurotrophic factor activity in neuronal cells. The compounds of this disclosure can be used to promote repair of neuronal damage caused by disease or physical trauma.

### Description of Related Art

The ability of neurons to extend neurites is of prime importance in establishing neuronal connections during development. It is also required during regeneration to re-establish connections destroyed as a result of a lesion.

One area that has been determined to be of significance in the repair and regeneration of cellular tissue, including neural tissue, is the extracellular matrix. During approximately the past two decades, the base knowledge of cell adhesion and migration in extracellular matrices (ECMs) at the molecular level has expanded rapidly. The action of enzymes and other polypeptides which degrade components of the extracellular matrix and basement membranes may facilitate the events of neural repair by a variety of mechanisms including the release of bound cytokines and by increasing the permeability of the matrix, thereby enhancing the mobility of mediator molecules, growth factors and chemotactic agents, as well as the cells involved in the healing process. For example, U.S. Patent No. 5,997,863 discloses the use of glycosaminoglycans to manipulate cell proliferation and promote wound healing.

While proteins, especially enzymes, may be used to remove the regeneration-inhibitory components of the extracellular matrix, their use alone may not be sufficient to effect repair of a damaged nervous system.

A number of molecules and specified regions thereof have been implicated in the ability to support the sprouting of neurites from a neuronal cell, a process also referred to as neurite outgrowth. This process is essential in neural development and regeneration, especially after physical injury or disease has damaged neuronal cells. Neurites elongate profusely during development both in the central and peripheral nervous systems of all animal species (Cajal, Degeneration and regeneration in nervous system, Oxford University Press, London (1928)). This phenomenon pertains to axons and dendrites. However, in adults, axonal and dendritic regrowth in the central nervous system is increasingly lost with evolutionary progression.

Various polypeptides, especially cell adhesion molecules (CAMs), have been known to promote neural cell growth. While early efforts in this area of research concentrated on the adhesion-promoting ECM protein fibronectin (FN), other polypeptides have also been found to promote neural growth. For example, U.S. patent No. 5,792,743, discloses novel polypeptides and methods for promoting neural growth in the central nervous system of a mammal by administering a soluble neural CAM, a fragment thereof, or a Fc-fusion product thereof. U.S. Patent No. 6,313,265 discloses synthetic polypeptides containing the pharmacologically active regions of CAMs that can be used in promoting nerve regeneration and repair in both peripheral nerve injuries as well as lesions in the central nervous system (CNS).

While helpful, the use of regenerative proteins alone may not be sufficient to effect repair of a damaged nervous system.

Chimeric proteins are known in the art as proteins derived from genetically different sources. They are commonly synthesized using recombinant DNA techniques. For example, U.S. Patent No. 6,326,166 discloses chimeric DNA-blinding proteins and methods for their manufacture and use and U.S. Patent No. 6,248,562 discloses chimeric proteins containing antigenic polypeptides derived from Lyme Disease causing strains of Borrelia.

WO 95/13091 discloses a chimeric protein comprising ECM-altering enzymic activity and an ECM-binding moiety. EP 0704532 discloses a chimeric protein having a matrix modifying polypeptide and a glycoprotein cellular regulatory factor.

### SUMMARY

In accordance with the present disclosure, a chimeric protein possessing at least two functional domains is disclosed. The functional domains code polypeptides that are not found together in nature but both of which promote regeneration and repair of damaged neural tissue.

In one embodiment, the first functional domain is for a polypeptide, which is a matrix digesting enzyme, possessing matrix modification activity by removal of components of the extracellular matrix that inhibit neural growth and development. The second functional domain is for a polypeptide known to possess neurotrophic factor activity for neural cells.

The disclosure extends to methods of promoting and enhancing neural regeneration in vivo utilizing chimeric proteins combining two or more domains coding for two or more therapeutic polypeptides, and to pharmaceutical compositions which may be used to accomplish the objectives of such methods. More specifically, the chimeric proteins of the present disclosure may be formulated in a composition that may be delivered to a neural site, as by parenteral administration.

The term "Chase" or "CHase" as used herein is an abbreviation for "chondroitinase".

### BRIEF DESCRIPTION OF THE FIGURES

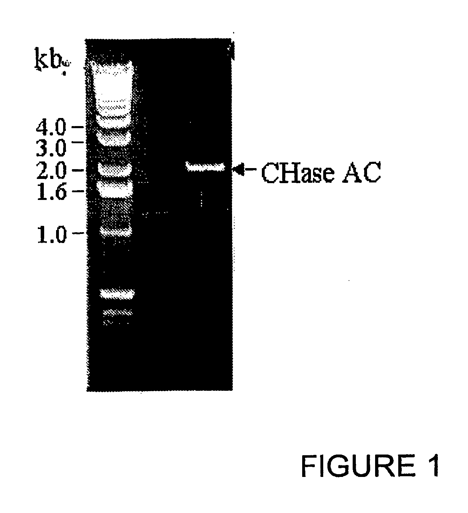

Fig. 1 is an agarose gel depicting the cloned fragment of Chase AC from Flavobacterium Heparinum.

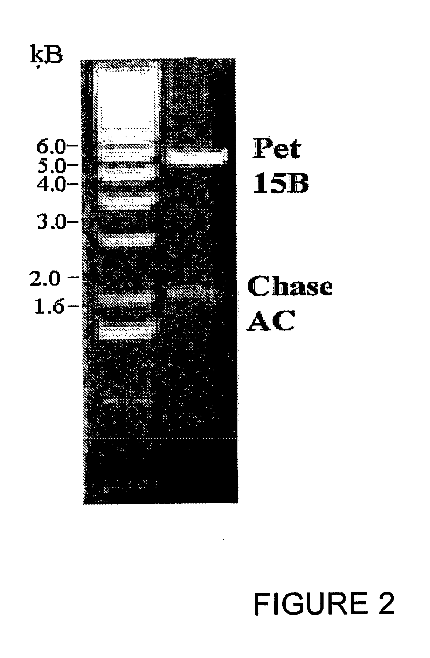

Fig. 2 shows the expression of Chondroitinase AC in Pet 15b vector.
Fig. 3 shows the protein expression of Chase AC.

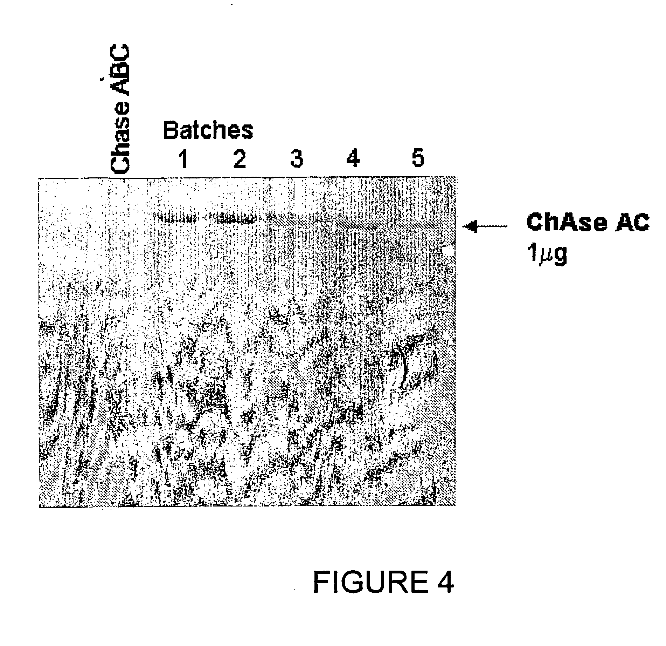

Fig. 4 is a zymography gel showing activity of recombinant Chase AC.

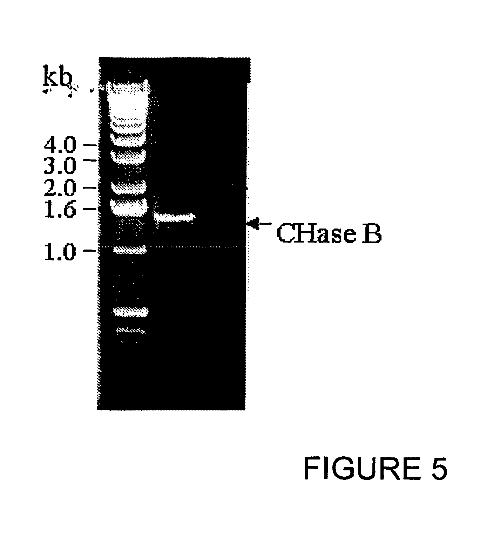

Fig. 5 is an agarose gel depicting the cloned fragment of Chase B from Flavobacterium Heparinum.

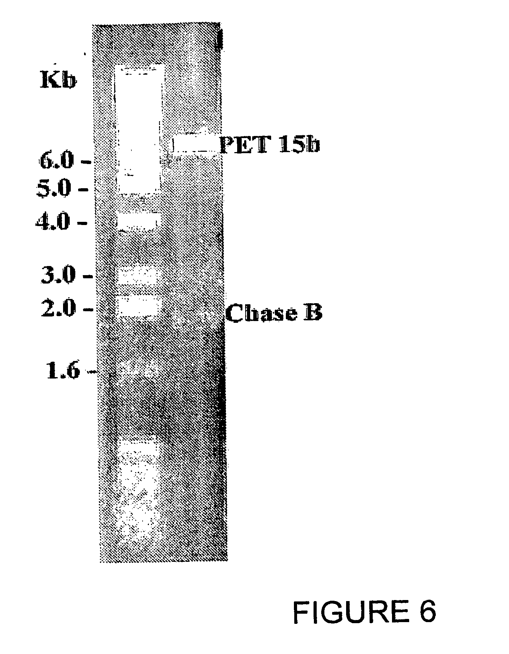

Fig. 6 shows the expression of Chondroitinase B in Pet 15b vector.
Fig. 7 shows the purification of Chase B using a Ni-NTA column and run on a 10% SDs-PAGE gel.

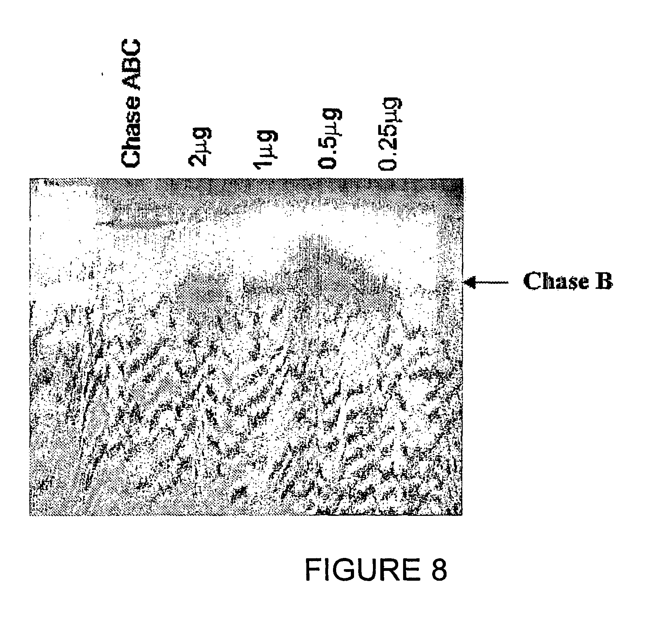

Fig. 8 is a zymography gel showing activity of recombinant Chase B.

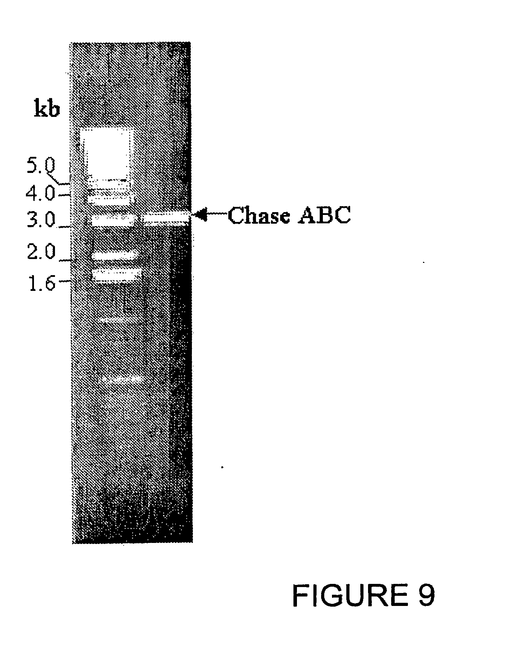

Fig. 9 is an agarose gel depicting cloned fragment of Chase ABC from Proteus Vulgaris.

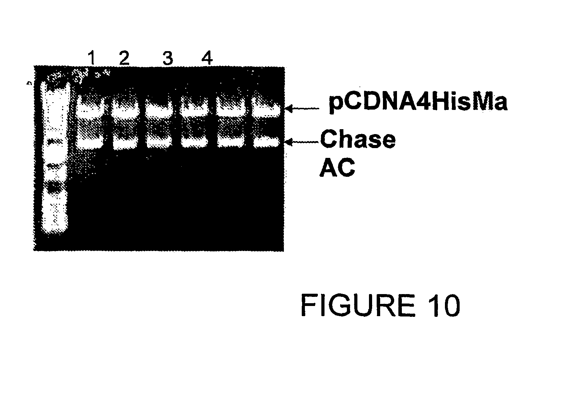

Fig. 10 is an agarose gel depicting expression of Chondroitinase AC in PCDNA4HisMax, a mammalian expression vector.
Fig. 11 shows Western blot showing Chase AC transfected CHO-K cell extracts.
Fig. 12 is an agarose gel depicting expression of Chondroitinase B in PCDNA4HisMax, a mammalian expression vector.
Fig. 13 shows a Western blot showing Chase B transfected CHO-K cell extracts.
Fig. 14 depicts a protein gel of purified human L1-Fc protein.
Fig. 15 shows the results of an outgrowth assay showing activity of human L1-Fc in promoting neurite outgrowth.

### DETAILED DESCRIPTION OF THE PREFERRED EMBODIMENTS

The current disclosure pertains to chimeric proteins combining polypeptides which do not occur together in nature. The chimeric proteins are a combination of one or more polypeptides capable of modifying the extracellular matrix as described above with at least one other polypeptide possessing neurotrophic factor activity for neural cells.

As used herein, the terms "protein", "polypeptide" and "peptide" are used interchangeably herein to designate a series of amino acid residues connected one to the other by peptide bonds between the alpha-amino and carboxy groups of adjacent residues.

The term "chimeric protein" describes a protein comprising two or more polypeptides which are derived from different species. A polypeptide "derived from" a species is a polypeptide which has an amino acid sequence the same as an amino acid sequence present in the species, an amino acid sequence equivalent to the amino acid sequence of a naturally occurring protein in a species, or an amino acid sequence substantially similar to the amino acid sequence of a naturally occurring protein in a species (e.g., differing by few amino acids) such as when a nucleic acid encoding a protein is subjected to site-directed mutagenesis.

"Corresponding" proteins are equivalent proteins from different species.

The chimeric proteins contain a first polypeptide and a second polypeptide as described above. Relevant polypeptides are combined into a single chimeric protein.

The chimer as described herein can be produced so that they are highly soluble, hyper-produced in E. coli, and non-lipidated. In addition, the chimeric proteins can be designed to end in an affinity tag (His-tag) to facilitate purification.

The chimeric proteins described herein can be produced by known techniques, such as by recombinant methodology, polymerase chain reaction (PCR), or mutagenesis. Chimeric proteins can also be formed by chemical crosslinking of the first protein to the second peptide or protein to form a chimeric protein. Numerous chemical crosslinking agents are known in the art (e.g., commercially available from Pierce, Rockford III.). A crosslinking agent can be chosen which allows for high yield coupling of the modulator to the second peptide or protein and for subsequent cleavage of the linker to release bioactive modulator. For example, a biotin-avidin-based linker system may be used.

In one embodiment, the two polypeptides of the chimeric protein are linked together by a peptide, such as a fragment of an immunoglobulin molecule. Thus, for example, the peptide linkage between the two polypeptides be an Fc fragment, which has been found to enhance the activity of the neurotrophic factor polypeptide component. In certain embodiments, the Fc fragment contains regions necessary to form disulfide-linked dimers, but does not include segments approaching the carboxyl terminus which tend to induce macrophage phagocytosis.

When recombinant techniques are employed to prepare a chimeric protein in accordance with this disclosure, nucleic acid (e.g., DNA) molecules or segments encoding the polypeptides are preferably used. The DNA molecules are then ligated (covalently bound) to a vector that is subsequently expressed in a suitable host.

Whenever an RNA molecule encoding one of the polypeptides of the present disclosure is used, the RNA molecule including the polypeptide coding molecule is transcribed into complementary DNA (cDNA) via a reverse transcriptase. The cDNA molecule can then be used as described herein to generate the subject polypeptide.

In a particularly useful embodiment, a DNA nucleotide sequence (molecule) encoding at least one of then amino acid residue sequences of the matrix modifying or neurotrophic factor polypeptide as described herein is operatively linked to a larger DNA molecule. The resultant DNA molecule is then transformed or transfected into a suitable host and expressed therein.

DNA segments (i.e., synthetic oligonucleotides) that encode the polypeptide components of the present disclosure can be synthesized by standard chemical techniques, for example, the phosphotriester method of Matteucci, et al., (J. Am. Chem. Soc., 103:3185-3191, 1981) or via automated synthesis methods. In addition, larger DNA segments can readily be prepared by well known methods, such as synthesis of a group of oligonucleotides that define the DNA segment, followed by hybridization and ligation of oligonucleotides to build the complete segment of the polypeptide of interest.

Of course, by chemically synthesizing the coding sequence, any desired modifications can be made simply by substituting the appropriate bases for those encoding the native amino acid residue sequence. Furthermore, DNA segments consisting essentially of structural genes encoding the polypeptide of interest can be obtained from recombinant DNA molecules containing a gene that defines a disclosed matrix modifying or regenerative peptide, and can be subsequently modified, as by site directed mutagenesis, to introduce the desired substitutions.

The polypeptide-encoding polynucleotide sequences formed in accordance with the present disclosure may also be produced by enzymatic techniques. Thus, restriction enzymes which cleave nucleic acid molecules at predefined recognition sequences can be used to isolate nucleic acid fragments from larger nucleic acid molecules containing the desired nucleic acid molecules such as the DNA (or RNA) that codes for one of the two components of the chimeric protein. Typically, DNA fragments produced in this manner will have cohesive, "overhanging" termini, in which single-stranded nucleic acid sequences extend beyond the double-stranded portion of the molecule. The presence of such cohesive termini is generally preferred over blunt-ended DNA molecules. The isolated fragments containing the desired coding sequence can then be ligated (cloned) into a suitable vector for amplification and expression.

PCR may also be used to synthesize the polypeptide-encoding polynucleotide sequences which may then be operatively linked to a vector and used to transform or transfect an appropriate cell and expressed therein. Particularly preferred methods for producing large quantities of recombinant polypeptides and proteins of the present invention rely on the use of preselected oligonucleotides as primers in a polymerase chain reaction (PCR) to form PCR reaction products for use in preparing expression vectors.

Expression of recombinant polypeptides and proteins in accordance with this disclosure is accomplished through the use of expression vectors into which the nucleotide sequences encoding the subject polypeptides have been inserted. The expression vectors may be constructed utilizing any of the well-known vector construction techniques.

The choice of vector to which a nucleotide segment is operatively linked depends directly, as is well known in the art, on the functional properties desired, e.g., protein expression, and the host cell to be transformed or transfected, these being limitations inherent in the art of constructing recombinant DNA molecules. However, a vector contemplated herein is at least capable of directing the replication, and preferably also expression, of the beneficial chimeric protein gene included in DNA segments to which it is operatively linked.

Thus, the present disclosure contemplates a vector that can be operatively linked to a nucleic acid molecule coding the polypeptide components of the chimeric protein to provide a recombinant DNA molecule that encodes and expresses the desired polypeptide sequence. The recombinant molecule can be used to transform or transfect suitable host cells so that the host cells express the desired chimeric protein.

In various embodiments, the translatable nucleotide sequence may be incorporated into a plasmid with an appropriate controllable transcriptional promoter, translational control sequences, and a polylinker to simplify insertion of the translatable nucleotide sequence in the correct orientation, and may be expressed in the host cells. Useful host cells include eukaryotic insect cells, such as Spodoptera frugiperda, or prokaryotic cells, such as Escherichia coli or mammalian cells, such as Chinese Hamster Ovary (CHO) cells. Preferably, there are 5' control sequences defining a promoter for initiating transcription and a ribosome binding site operatively linked at the 5' terminus of the upstream translatable DNA sequence. Examples of useful expression vectors including promoters such as tac, trc, or PL, for example, include pTrc99A (Pharmacia, Piscataway, N.J.), pKK223-3 (Clontech), and PET 3d (Novagen).

Transformation or transfection of appropriate cell hosts with a recombinant DNA molecule is accomplished by well known methods that typically depend on the type of vector used. With regard to transformation of prokaryotic host cells, see, for example, Maniatis et al., Molecular Cloning, A Laboratory Manual, Cold Spring Harbor Laboratory, Cold Spring Harbor, N.Y. (1982).

Successfully transformed or transfected cells, i.e., those containing a recombinant DNA molecule in accordance with the present disclosure, can be identified by well known techniques. For example, transformed or transfected cells can be cloned to produce monoclonal colonies. Cells from those colonies can be harvested, lysed and their DNA content examined for the presence of the desired DNA molecule using a method such as that described by Southern, J. Mol. Biol., 98:503 (1975).

In addition to directly assaying for the presence of the desired DNA molecule, successful transformation or transfection can be confirmed by well known methods for detection of the expressed polypeptides. For example, cells transformed or transfected with an expression vector produce proteins that can be harvested and assayed for the presence of the function of the expressed protein.

Methods for recovering an expressed protein from a culture are well known in the art. For instance, gel filtration, gel chromatography, ultrafiltration, electrophoresis, ion exchange, affinity chromatography and related techniques can be used to isolate the expressed proteins found in the culture. In addition, immunochemical methods, such as immunoaffinity, immunoadsorption, and the like, can be performed using well known methods.

The use of multiple therapeutic agents is an approach that can be utilized in which the benefit of the first polypeptide's removal of the inhibitory components of the matrix can be augmented by the use of a second polypeptide which stimulates neural repair and regeneration. The polypeptides of the present disclosure are preferably selected from a group of matrix-digesting enzymes and a second group of cell adhesion molecules having regenerative properties for CNS tissue. The genes encoding these proteins may be combined through genetic engineering to produce a chimeric gene capable of producing a chimeric protein possessing both the matrix-digesting enzyme and the cell adhesion molecule.

As described above, the first component of the chimeric protein is a polypeptide possessing the ability to modify the extracellular matrix as described, and is an enzyme capable of digesting components of the extracellular matrix that inhibit cell growth. Such enzymes include, but are not limited to, chondroidnase, hyaluronidase, and other proteinases, such as matrix metalloproteinases (MMP's), MMP-9, MMP-2, pepsin, cathepsin D, and the ADMAT family of proteins etc.

In one embodiment, the enzymatic compound is a chondroitinase enzyme produced by bacteria. The chondroitinases function by degrading polysaccharide side chains in protein-polysaccharide complexes, without degrading the protein core. The enzyme selectively degrades the glycosaminoglycans chondroitin-4-sulfate, dermatan sulfate and chondroitin-6-sulfate (also referred to respectively as chondroitin sulfates A, B and C) at pH 8 at higher rates than chondroitin or hyaluronic acid. However, the enzyme does not attack keratan sulfate, heparin or heparitin sulfate.

Recent evidence suggests that chondroitin sulfate proteoglycan (CSPG) plays a major role in the inhibition of regeneration of a damaged central nervous system (CNS). It has been found that CSPG is upregulated in damaged CNS, further exacerbating its inhibitory effect. Recent evidence also suggests that chondroitinase ABC treatment of CNS lesions can improve repair and regeneration of a damaged CNS.

In particular, the chondroitinase ABC produced by Proteus vulgaris is considered to be appropriate to medical and commercial applications because of its capability of selectively removing side chain of chondroitin sulfate or dermatan sulfate from proteoglycan, its inactivity toward keratan sulfate, heparin, and heparan sulfate, and its abundant productivity. Because of this, enzyme preparations having chondritinase activity are prepared from culture products of Proteus vulgaris by processes known to those skilled in the art.

Other components of the CNS matrix, such as hyaluronic acid and other proteins, may also play a role in inhibiting neural regeneration. Accordingly, hyaluronidase, a bacterial enzyme known to degrade hyaluronic acid, and matrix metalloproteinases, may also be used as the enzymatic component of the chimeric protein to remove compounds of the extracellular matrix that impede cell repair and regeneration.

With respect to the second component of the disclosed chimeric protein, members of the neurotrophic family of growth factors are used, such as Nerve Growth Factor (NGF), Neurotrophin-3 (NT-3) and Brain Derived Neurotrophic Factor (BDNF) among others. These molecules provide for neuronal survival during development by preventing cell death and encouraging neuronal outgrowth in a targeted fashion as they are secreted by some of the natural targets of developing neuron. In addition, neurotrophic factors have been used to augment the poor intrinsic regenerative capacity of central nervous system neurons in various fashions (Priestley et al., J Physiol Paris, 96:123-33(2002)).

In selecting a particular subject chimeric protein, any of the polypeptides described herein can be utilized to form the chimeric protein and to promote repair, irrespective of the species of neuronal cell and species of protein from which a subject polypeptide is derived.

The chimeric proteins of the present disclosure may be used in improving nerve regeneration or promoting nerve repair and survival, and may also fmd use in treating peripheral nerve injury and spinal cord injury, and in stimulation of growth of endogenous, implanted or transplanted CNS tissue. The chimeric proteins of the current invention are particularly advantageous when used with injuries to the central nervous system in that the regenerative peptide component retains its affinity for the CNS and thus imparts a targeting function directing the enzyme component to the CNS. The co-localization of the two components increases their effectiveness compared to independent use.

The present disclosure therefore also provides chimeric proteins as described above for promoting regeneration of an injured or severed nerve or nerve tissue, or promoting neurite outgrowth in neuronal cells under a variety of neurological conditions requiring neuronal cell outgrowth. Thus the present disclosure contemplates promoting nerve regeneration and repair in a subject wherein a physiologically tolerable pharmaceutical composition containing a therapeutically effective amount of a chimeric protein in accordance with the present disclosure is administered to the subject or tissue.

The present therapeutic proteins are useful in treating peripheral nerve damage associated with physical or surgical trauma, infarction, bacterial or viral infection, toxin exposure, degenerative disease, malignant disease that affects peripheral or central neurons, or in surgical or transplantation methods in which new neuronal cells from brain, spinal cord or dorsal root ganglia are introduced and require stimulation of neurite outgrowth from the implant and innervation into the recipient tissue. Such diseases further include but are not limited to CNS lesions, gliosis, Parkinson's disease, Alzheimer's disease, neuronal degeneration, and the like.

In a related embodiment, a polypeptide of the invention can be impregnated into an implantable delivery device such as a cellulose bridge, suture, sling prosthesis or related delivery apparatus. Such a device can optionally be covered with glia, as described by Silver, et al, Science 220:1067-1069, (1983)_{.}

The composition containing the chimeric protein may also be incorporated or impregnated into a bioabsorbable matrix, with the matrix being administered in the form of a suspension of matrix, a gel or a solid support. In addition, the matrix may be comprised of a biopolymer.

In constructing the matrix, it may be useful for the matrix to further include a substructure for purposes of administration and/or stability. Suitable substructures include freeze dried sponge, powders, films, flaked or broken films, aggregates, microspheres, fibers, fiber bundles, or a combination thereof.

In addition, the matrix may be attached to a solid support for administration purposes. Suitable supports depend upon the specific use and can include a prosthetic device, a porous tissue culture insert, an implant, a suture, and the like.

Therapeutic compositions of the present disclosure may include a physiologically tolerable carrier together with at least one chimeric protein of this invention as described herein, dispersed therein as an active ingredient. In a preferred embodiment, the therapeutic composition is not immunogenic when administered to a human patient for therapeutic purposes.

As used herein, the terms "pharmaceutically acceptable", "physiologically tolerable" and grammatical variations thereof, as they refer to compositions, carriers, diluents and reagents, are used interchangeably and represent that the materials are capable of administration upon a mammal or human without the production of undesirable physiological effects such as nausea, dizziness, gastric upset and the like.

The preparation of a pharmacological composition that contains active ingredients dispersed therein is well understood in the art. Typically such compositions are prepared as sterile compositions either as liquid solutions or suspensions, aqueous or non-aqueous, however, suspensions in liquid prior to use can also be prepared.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

A therapeutic composition of the present invention can include pharmaceutically acceptable salts of the components therein. Pharmaceutically acceptable salts include the acid addition salts (formed with the free amino groups of the chimeric protein) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.

Effective amounts can be measured by improvements in neuronal or ganglion cell survival, axonal regrowth, and connectivity using well known methods. See, e.g., Bray, et al., "Neuronal and Nonneuronal Influences on Retinal Ganglion Cell Survival, Axonal Regrowth, and Connectivity After Axotomy", Ann. N.Y. Acad. Sci., pp. 214-228 (1991). Improvements in neuronal regeneration in the CNS and PNS are also indicators of the effectiveness of treatment with the disclosed compounds and compositions, as are improvements in nerve fiber regeneration following traumatic lesions. (See, e.g., Cadelli, et al., Exp. Neurol. 115: 189-192 (1992), and Schwab, Phil. Trans. R. Soc. Lond. 331: 303-306 (1991).)

Thus, the dosage ranges for the administration of a chimeric protein of the invention are those large enough to produce the desired effect in which the condition to be treated is ameliorated. The dosage should not be so large as to cause adverse side effects. Generally, the dosage will vary with the age, condition, and sex of the patient, and the extent of the disease in the patient, and can be determined by one of skill in the art. The dosage can be adjusted by the individual physician in the event of any complication.

The compositions are administered in a manner compatible with the dosage formulation, and in a therapeutically effective amount. A therapeutic amount of a chimeric protein of this invention is an amount sufficient to produce the desired result, and can vary widely depending upon the disease condition and the potency of the therapeutic compound. The quantity to be administered depends on the subject to be treated, the capacity of the subject's system to utilize the active ingredient, and the degree of therapeutic effect desired. Precise amounts of active ingredient required to be administered depend on the judgment of the practitioner and are peculiar to each individual. However, suitable dosage ranges for systemic application are disclosed herein and depend on the conditions of administration. Suitable regimes for administration are also variable, but are typified by an initial administration followed by repeated doses at one or more hour intervals by a subsequent administration.

A therapeutically effective amount of a chimeric protein is typically an amount such that when it is administered in a physiologically tolerable composition, it is sufficient to achieve a plasma or local concentration of from about 0.1 to 1,000 micromolar (µm), preferably about 1 to 100 µm.

Alternatively, the dosage can be metered in terms of the body weight of the patient to be treated. In this case, a typical dosage of a therapeutic composition is formulated to deliver a pharmacologically active polypeptide of this invention is amount of about 0.1 microgram (µg) to 100 µg per kilogram (kg) body weight, or more preferably about 1 to 50 µg/kg.

A chimeric protein can be administered parenterally by injection or by gradual infusion over time. For example, a polypeptide of the invention can be administered topically, locally, perilesionally, perineuronally, intracranially, intravenously, intrathecally, intramuscularly, subcutaneously, intracavity, transdermally, dermally, or via an implanted device, and they may also be delivered by peristaltic means. In general, local, perilesional, intrathecal, perineuronal, or intra-CNS administration is preferred.

The therapeutic compositions containing a chimeric protein are conventionally administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

Alternatively, continuous intravenous infusion sufficient to maintain therapeutically effective concentrations in the blood are contemplated. Therapeutically effective blood concentrations of a chimeric protein are in the range of about 0.01 □ to about 100 □, preferably about 1 □ to about 10 □.

The terms "therapeutically effective" or "effective", as used herein, may be used interchangeably and refer to an amount of a therapeutic composition of the present invention-e.g., one containing a chimeric protein of this disclosure. For example, a therapeutically effective amount of a chimeric protein containing composition, or beneficial compound therein, is a predetermined amount calculated to achieve the desired effect, i.e., to effectively promote neural repair and regeneration, including neurite outgrowth of neurons in an individual to whom the composition is administered.

### EXAMPLE 1

### Cloning of chondroitinase AC from Flavobacterium heparinum:

Flavobacterium heparinum (ATCC) was grown in LB (Luria broth) at 25°C for 4 days. The bacteria were spun down by centrifugation and genomic DNA was isolated by DNeasy Tissue kit (Qiagen). PCR primers were synthesized (Pojasek et. al (2001), Biochem Biophys. Res Com, 286, 343-351) with a NdeI restriction site at the 5' end and a BamHI site at the 3' end having sequences 5'-CATATGCAGCAGACCGGTACTGCA-3' (Seq. ID No. 1) and 5'-GGATTCTCAGTGCTCTTTATTTCT-3' (Seq. ID No. 2) respectively to synthesize the mature protein. One microgram of the genomic DNA was used in a 50 µl PCR reaction containing 10 mM of each dNTP (dATP, dTTP, dCTP and dGTP), 50 pmol each of forward and reverse primers, 1 mM of MgSO₄, and 5 units of Tfl DNA polymerase (Promega). The 2.0 kb PCR product was ligated into pCR 2.1 vector (TOPO cloning kit, Invitrogen) and transformed into OneShot competent cells (Invitrogen). Plasmid DNA was isolated from a number of clones screened by digestion with EcoRI restriction enzyme and the positive clones selected having the 2.0 kb insert. The genes were further cloned into pET 15b (Novagen) at the NdeI and BamHI sites and confirmed by DNA sequencing.

### Expression and purification of chondroitinase AC:

The plasmid DNA containing the chondroitinase AC in pET15b was transformed in BL21(DE3) for expression. Bacterial cultures in LB medium were grown to O.D. 0.6 and induced with 1 □M IPTG and induction continued overnight at 22°C. The cells were harvested at 5000 rpm for 15 min and resuspended in buffer A (1/20 of the initial culture volume) containing 20 mM Tris (pH 7.9), 500 mM NaCl, 5 mM imidazole. The resuspended cells were lysed by sonication on ice. The soluble protein was isolated by centrifugation at 13,000 rpm for 20 min at 4°C. An Ni-NTA (Qiagen) column (2-ml) was equilibrated with buffer A and the supernatant loaded onto it. The column was washed thoroughly with at least 30-50 column volumes of buffer A, until the A280 reached 0.002 or lower. The bound protein was eluted by buffer A containing 250 mM imidazole. The fractions containing the ChaseAC were analyzed on 10% SDS-PAGE gels and pooled. The functional activity was tested by zymography using aggrecan as a substrate.

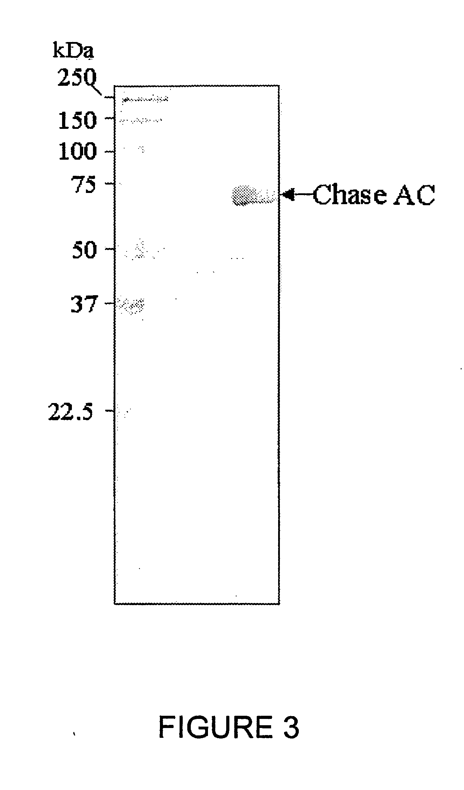

Chase AC was run on a 10% non-denaturing SDS-PAGE gel followed by 16 h renaturation at 370°C. The gel was stained with Alcian blue which stains carbohydrates, enzymatic activity was seen as a lack of Alcian blue staining (depicted in Fig. 3 as dark grey bands).

### EXAMPLE 2

### Cloning of chondroitinase B from Flavobacterium heparinum:

Similarly, chondroitinase B was amplified as above using the primers with a Ndel restriction site at the 5' end and a BamHI site at the 3' end having sequences 5'-CATATGCAGGTTGTTGCTTCAAAT-3' (Seq. ID No. 3) and 5'-GATCCTCAGTGCTCTTTATTTCT-3' (Seq. ID No. 4) respectively to synthesize the mature protein. One microgram of the genomic DNA was used in a 50 µl PCR reaction containing 10 mM of each dNTP (dATP, dTTP, dCTP and dGTP), 50 pmol each of forward and reverse primers, 1 mM of MgSO₄, and 5 units of Tf1 DNA polymerase (Promega). The 1.5 kb PCR product was ligated into pCR 2.1 vector (TOPO cloning kit, Invitrogen) and transformed into OneShot competent cells (Invitrogen). Plasmid DNA was isolated from a number of clones screened by digestion with EcoRI restriction enzyme and the positive clones selected having the 1.5 kb insert. The genes were further cloned into pET 15b (Novagen) at the Ndel and BamHI sites and confirmed by DNA sequencing.

### Expression and purification of chondroitinase B:

The plasmid DNA containing the chondroitinase B in pET15b was transformed into BL21(DE3) for expression. Bacterial cultures in LB medium were grown to O.D. 0.6 and induced with 1 mM IPTG and induction was continued overnight at 22°C. The cells were harvested at 5000 rpm for 15 min and resuspended in buffer A (1/20 of the initial culture volume) containing 20 mM Tris (pH 7.9) and 500 mM NaCl (Buffer A). The resuspended cells were lysed by sonication on ice. The soluble protein was isolated by centrifugation at 13,000 rpm for 20 min at 4°C. An Ni-NTA (Qiagen) column (2-ml) was equilibrated with buffer A and the supernatant loaded onto it. The column was washed thoroughly with at least 30-50 column volumes of buffer A, until the A280 reached 0.002 or lower. The bound protein was eluted by buffer A containing 250 mM imidazole. The fractions containing the Chondroitinase B were analyzed by running on 10% SDS-PAGE gels and the fractions were pooled. The functional activity was tested by zymography using aggrecan as a substrate.

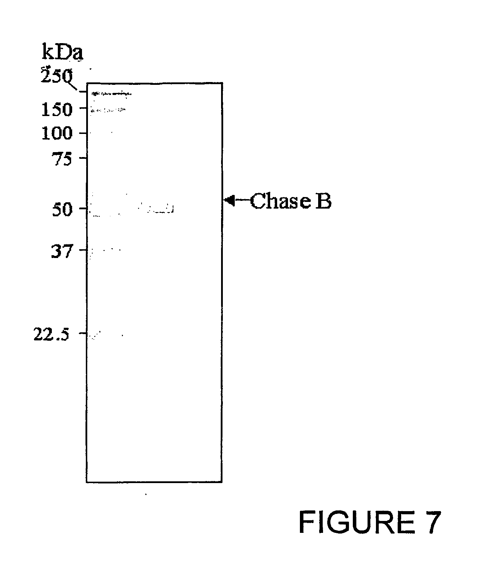

Chase B was run on a 10%a non-denaturing SDS-PAGE gel followed by 16 h renaturation at 370°C. The gel was stained with Alcian blue which stains carbohydrates, enzymatic activity is seen as a lack of Alcian blue staining (depicted in Fig. 7 as dark grey bands).

### EXAMPLE 3

### Cloning of chondroitinase ABC from Proteus vulgaris:

Genomic DNA was isolated from Proteus vulgaris using DNeasy Tissue kit (Qiagen). PCR primers were synthesized with an NdeI restriction site at the 5' end and a BamHI site at the 3' end having sequences 5'- CAT ATG GCC ACC AGC AAT CCT GCA TTT G-3'(Seq. ID No. 5) and 5'- GGA TCC TCA AGG GAG TGG CGA GAG-3' (Seq. ID No. 6), respectively. The 3.0 kb PCR product was ligated into pCR 2.1 vector (TOPO cloning kit, Invitrogen) and transformed into OneShot competent cells (Invitrogen). Plasmid DNA was isolated from a number of clones screened by digestion with EcoRI restriction enzyme and the positive clones selected having the 3.0 kb insert. The genes were further cloned in pET 15b (Novagen) at the NdeI and BamHI sites and confirmed by DNA sequencing.

### Expression and purification of chondroitinase ABC:

The plasmid DNA containing the chondroitinase ABC in pET15b is transformed in BL21(DE3) for expression. Bacterial cultures in LB medium are grown to O.D. 0.6, induced with 1 mM IPTG and induction is continued overnight at 22°C. The cells are harvested at 5000 rpm for 15 min and are resuspended in buffer A (1/20 of the initial culture volume) containing 20 mM Tris (pH 7.9) and 500 mM NaCl (Buffer A). The resuspended cells are lysed by sonication on ice. The soluble protein is isolated by centrifugation at 13,000 rpm for 20 min at 4°C. An Ni-NTA (Qiagen) column (2-ml) is equilibrated with buffer A and the supernatant is loaded onto it. The column is washed thoroughly with at least 30-50 column volumes of buffer A, until the A280 reaches 0.002 or lower. The bound protein was eluted by buffer A containing 250 mM imidazole. The fractions containing the chondroitinase ABC are analyzed by 10% SDS-PAGE electrophoresis and are pooled. The functional activity is tested by zymography using aggrecan as a substrate.

### Cloning and expression of Chase AC in pCDNA4HisMax a mammalian expression vector:

PCR primers were synthesized with a BamHI restriction site at the 5' end and an EcoRI site at the 3' end having sequences 5'-GGATCCCAGCAGACCGGTACTGCA-3' (Seq. ID No. 7) and 5'-GAATTCTCAGTGCTCTTTATTTCT-3' (Seq. ID No. 8) respectively to synthesize the mature protein. One microgram of the Chase AC DNA in pCR2.1 was used in a 50 µl PCR reaction containing 10 mM of each dNTP (dATP, dTTp, dCTP and dGTP), 50 pmol each of forward and reverse primers, 1 mM of MgSO₄, and 5 units of Tfl DNA polymerase (Promega). The 2.0 kb PCR product was ligated into pCR 2.1 vector (TOPO cloning kit, Invitrogen) and transformed into OneShot competent cells (Invitrogen). Plasmid DNA was isolated from a number of clones screened by digestion with EcoRI restriction enzyme and the positive clones are selected having the 2.0 kb insert. The gene was further cloned in pCDNA4HisMax at the BamHI and EcoRI sites and confirmed by DNA sequencing.

### Transfection of CHO-K cells with Chase AC in pCDNA4HisMax vector:

CHO-K cells were plated at a density of 1 x 10⁶ per well in a 6-well plate so that they are almost 90% confluent after 18h. The cells were tranfected with 1µg/ml of plasmid DNA purified by Qiagen DNA purification kit (Qiagen) using Lipofectamin 2000 reagent (Invitrogen). The transfected CHO cells were plated at a higher dilution and were selected against 400 µg/ml of zeocin (Invitrogen) starting at 48 hours after transfection. The individual clones resistant to zeocin were expanded in 48-well plates. The cells from each individual clones were harvested and whole cell extract was prepared using M-Per (Pierce) lysis buffer following manufacturer's protocol. The transfected or untransfected CHO extracts were resolved in 4-20% gradient SDS-PAGE and were transferred to nitrocellulose membrane followed by probing with HRP labeled anti- His antibody and detection by chemiluminescence (ECL kit, Amersham-Pharmacia. The positive clones are screened for activity by zymography gel assay using aggrecan as a substrate as before.

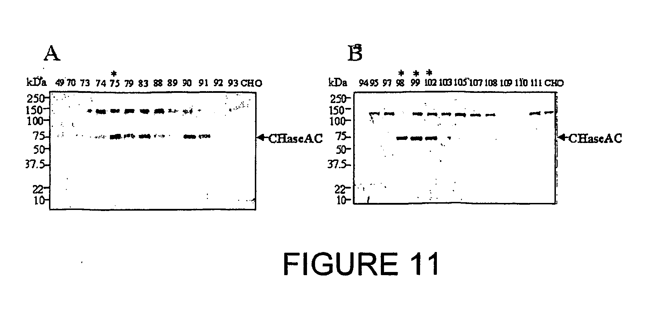

Figure 11 contains Western blot showing transfected CHO-K cell extracts. Extracts were run on polyacrylamide gels, proteins were transferred to nitrocellulose membranes and probed with an antibody against the Histidine-tagged Chase AC proteins. Asterisks denote higher expressing clones selected for further purification.

### Cloning of Chase B in pCDNA4HisMax, a mammalian expression vector.

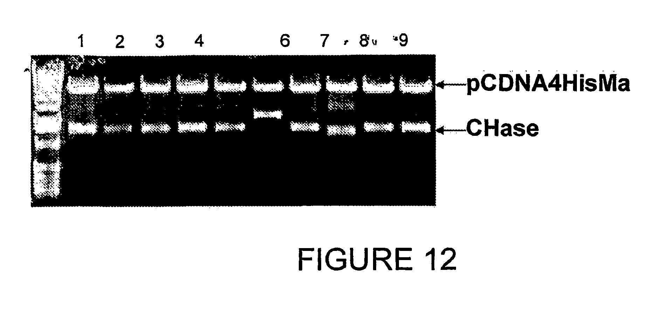

Similarly, Chase B was cloned in pCDNA4HisMax vector at the BamHI and EcoRI sites. PCR primers were synthesized with a BamHI restriction site at the 5' end and an EcoRI site at the 3' end having sequences 5'-GGATCCCAGGTTGTTGCTTCAAAT-3' (Seq. ID No. 9) and 5'GAATTCTCAGTGCTCTTTATTTCT-3' (Seq. ID No. 10) respectively to synthesize the mature protein. One microgram of the Chase B DNA in pCR2.1 was used in a 50 µl PCR reaction containing 10 mM of each dNTP (dATP, dTTP, dCTP and dGTP), 50 pmol each of forward and reverse primers, 1 mM of MgSO₄, and 5 units of Tf1 DNA polymerase (Promega). The 1.6 kb PCR product was ligated into pCR 2.1 vector (TOPO cloning kit, Invitrogen) and transformed into OneShot competent cells (Invitrogen). Plasmid DNA was isolated from a number of clones screened by digestion with EcoRI restriction enzyme and the positive clones selected having the 1.6 kb insert. The gene was further cloned in pCDNA4HisMax at the BamHI and EcoRI sites and confirmed by DNA sequencing. The DNA was purified by HiSpeed plasmid midi-kit (Qiagen) and was transfected into CHO cells by Lipofectamin2000 (Invitrogen) following the manufacturer's protocol. Figure 12 is an agarose gel depicting expression of Chondroitinase B in PCDNA4HisMax, a mammalian expression vector

### Transfection of CHO-K cells with Chase B in pCDNA4HisMax vector:

CHO-K cells were plated at a density of 1 x 10⁶ per well in a 6-well plate so that they are almost 90% confluent after 18h. The cells were tranfected with 1µg/ml of plasmid DNA purified by Qiagen DNA purification kit (Qiagen) using Lipofectamin 2000 reagent (Invitrogen). The transfected CHO cells were plated at a higher dilution and were selected against 400 µg/ml of zeocin (Invitrogen) starting at 48 hours after transfection. The individual clones resistant to zeocin were expanded in 48-well plates. The cells from each individual clones were harvested and whole cell extract was prepared using M-Per (Pierce) lysis buffer following manufacturer's protocol. The transfected or untransfected CHO extracts were resolved in 4-20% gradient SDS-PAGE and were transferred to nitrocellulose membrane followed by probing with HRP labeled anti- His antibody and detection by chemiluminascence (ECL kit, Amersham-Phannacia. The positive clones are screened for activity by zymography gel assay using aggrecan as a substrate as before.

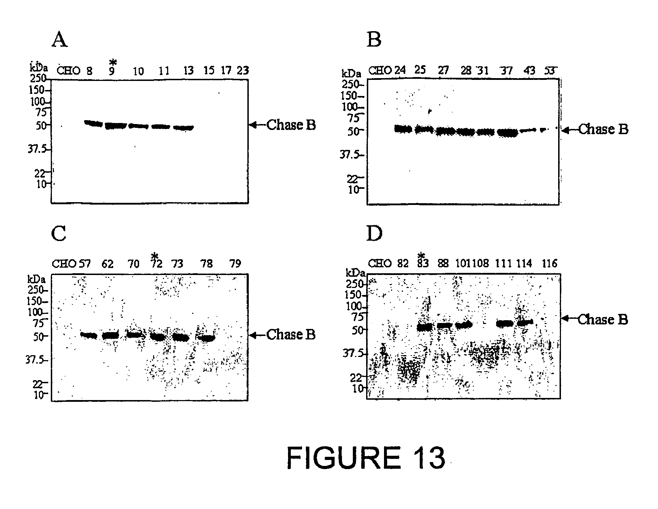

Figure 13 is a Western blot showing transfected CHO-K cell extracts. Extracts were run on polyacrylamide gels, proteins were transferred to nitrocellulose membranes and probed with an antibody against the Histidine-tagged Chase B proteins. Asterisks denote higher expressing clones selected for further purification.

### Cloning of Chase ABC in pCDNA4HisMax, a mammalian expression vector:

Chase ABC is cloned in pCDNA4HisMax vector at the BamHI and EcoRI sites. PCR primers are synthesized with a BamHI restriction site at the 5' end and an EcoRI site at the 3' end having sequences 5'- GGA TTC GCC ACC AGC AAT CCT GCA TTT G-3' and 5'- GAA TTC TCA AGG GAG TGG CGA GAG-3' respectively. One microgram of the Chase ABC DNA in pCR2.1 is used in a 50 µl PCR reaction containing 10 mM of each dNTP (dATP, dTTP, dCTP and dGTP), 50 pmol each of forward and reverse primers, 1 mM of MgSO₄, and 5 units of Tf1 DNA polymerase (Promega). The 3.0 kb PCR product is ligated into pCR 2.1 vector (TOPO cloning kit, Invitrogen) and transformed into OneShot competent cells (Invitrogen)). Plasmid DNA is isolated from a number of clones screened by digestion with EcoRI restriction enzyme and the positive clones are selected having the 3.0 kb insert. The gene is further cloned in pCDNA4HisMax at the BamHI and EcoRI sites and confirmed by DNA sequencing.

### Transfection of CHO-K cells with Chase ABC in pCDNA4HisMax vector

CHO-K cells are plated at a density of 1 x 10⁶ per well in a 6-well plate so that they are almost 90% confluent after 18h. The cells are tranfected with 1µg/ml of plasmid DNA purified by Qiagen DNA purification kit (Qiagen) using Lipofectamin 2000 reagent (Invitrogen). The transfected CHO cells are plated at a higher dilution and selected against 400 µg/ml of zeocin (Invitrogen) starting at 48 hours after transfection. The individual clones resistant to zeocin are expanded in 48-well plates. The cells from each individual clones are harvested and whole cell extract prepared using M-Per (Pierce) lysis buffer following manufacturer's protocol. The transfected or untransfected CHO extracts are resolved in 4-20% gradient SDS-PAGE and are transferred to nitrocellulose membrane followed by probing with HRP labeled anti-His antibody and detection by chemiluminascence (ECL kit, Amersham-Pharmacia). The positive clones are screened for activity by zymography gel assay using aggrecan as a substrate as before.

### Purification of L1 protein: (Comparative)

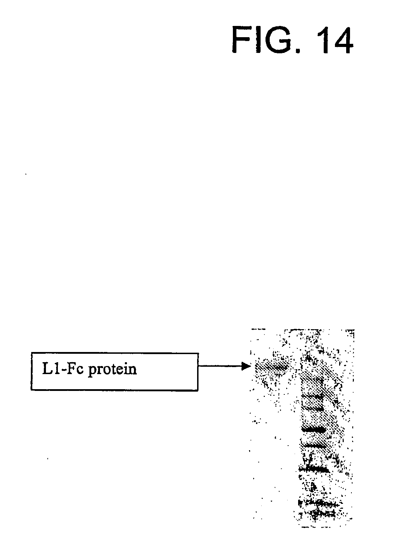

Conditioned media from L1 expressing cells was spun at 3000 * g for 60 minuntes at 4°C and the media filtered using a 40µm filter. Filtered conditioned media was diluted with deionised water and pHed to 6.4. Conditioned media was then run on a DE-52 column and eluted with 17.5 mM sodium phosphate plus 0.5 M sodium chloride at pH 6.4 following washing. Fractions from the column run were tested by Human IgG-Fc capture Elisa and Protein A slurry added to fractions showing a positive reaction. Eluate was incubated with protein A beads overnight at 4°C . The beads were separated by centrifugation, washed extensively, and L1-Fc eluted using 100mM glycine pH 2.8. (See Fig. 14). A further human IgG-Fc capture elisa was performed, and fractions containing protein were dialysed against PBS pH 6.4 and sterilized. Activity was confirmed by plating cerebellar granule neurons on L1 and comparing neurite length to neurons grown on poly-1-lysine.

### Outgrowh assay showing activity of L1 in promoting neurite outgrowth: (Comparative)

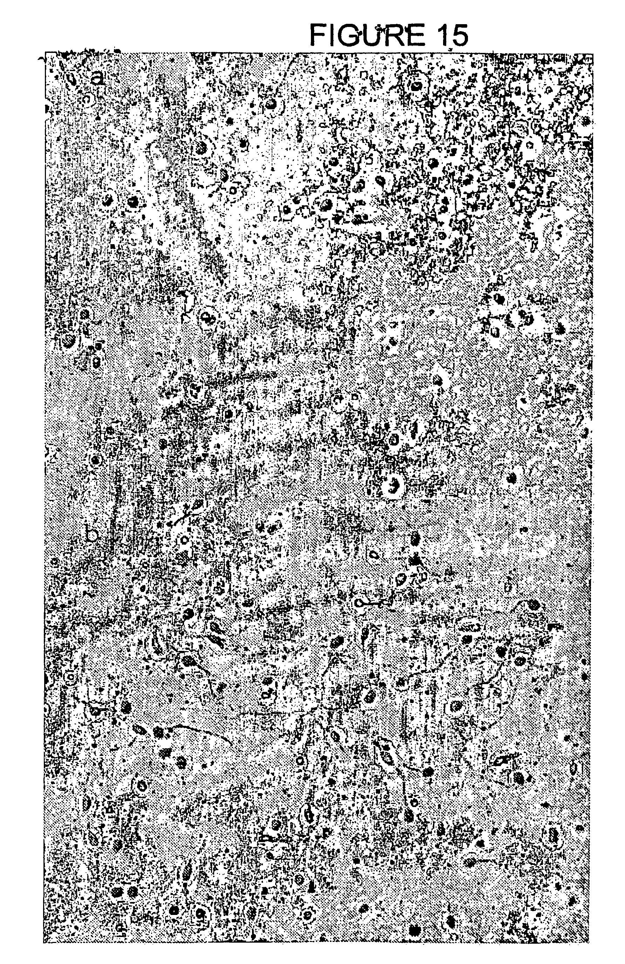

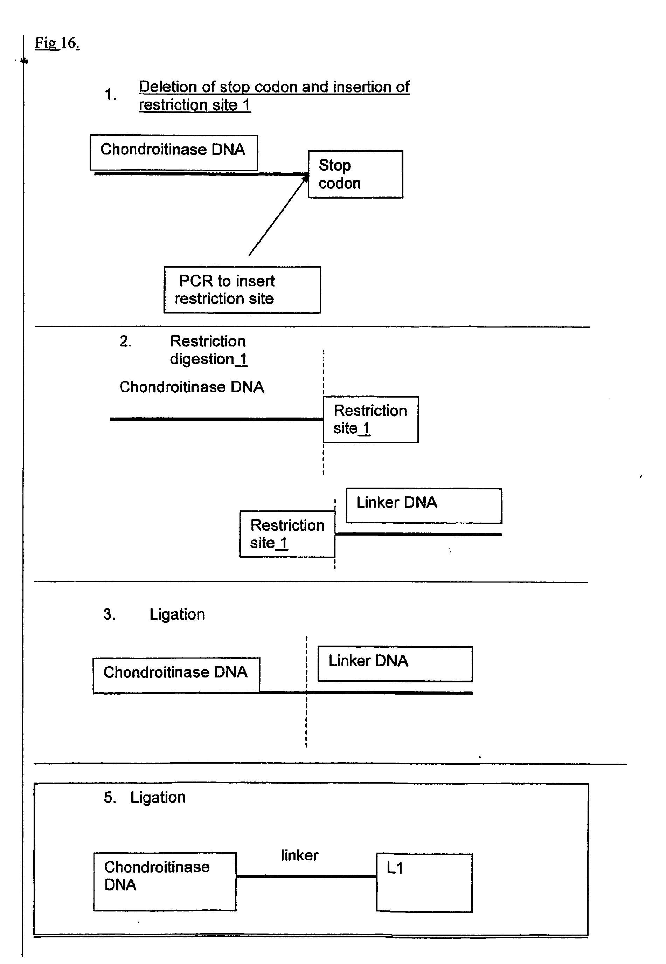

As seen in Fig. 15, neuritic processes eminating from cerebellar granule neurons grown on poly-lysine (a) are approximately 200% shorter than the neuritic processes that extend from neurons grown on L1 (b).

### Production of chimeric protein:

For the expression of the chimeric proteins, the DNA encoding chondroitinase enzyme is prepared by standard PCR from PCDNA4 as above. In order to produce the chimeric protein, primers are prepared to include a restriction site, such as BamHl just prior to the stop codon of the chondroitinase enzyme. A polyamino acid linker molecule, such as (Gly4Ser)3 (Kim, et al., J Biol Chem, 269, pages 31978-8 (1994)) is prepared by standard PCR using a primer which inserts the same restriction site at 5' of the DNA sequence encoding the linker molecule. DNA is amplified by standard PCR, digested with the appropriate restriction enzyme, and the two strands are joined together by standard ligation methods.

An alternate method of producing the fusion molecule of chondroitinase and the polyamino acid linker, is to prepare a mismatch primer which will change the DNA encoding the stop codon of the chondroitinase into an amino acid encoding the first of the amino acids in the linker molecule, followed by insertion of a restriction site. Recombinants are screened by starndard restriction digestion analysis. Recombinants are again screened by standard restriction digestion analysis, sequence confirmed by dideoxysequencing, and cloned into an expression vector to generate an in frame fusion protein. Recombinant protein is then expressed and purified as above, and the molecule tested for matrix modification activity and neurite outgrowth promoting activity as above.

Although the foregoing methods, compounds and compositions have been described in some detail by way of illustration and example for purposes of clarity of understanding, it will be apparent to one skilled in the art that certain changes and modifications may be practiced without departing from the essential characteristics of the invention.

## Claims

1. A chimeric protein comprising:
1) a first polypeptide possessing matrix modification activity by removal of components of the extracellular matrix which inhibit neuronal growth and development and which is selected from matrix digesting enzymes; and
2) a second polypeptide possessing neurotrophic factor activity for neural cells,
said first and second polypeptides not occurring together in nature and being joined together in said chimeric protein.

2. The chimeric protein of Claim 1 wherein the first polypeptide is selected from the group consisting of chondroitinases, hyaluronidases, and matrix metalloproteinases.

3. The chimeric protein of Claim 2 wherein the chondroitinase is selected from the group consisting of chondroitinase ABC exolyase, chondroitinase ABC endolyase, chondroitinase AC, and chondroitinase B.

4. The chimeric protein of Claim 2 wherein the chondroitinase is selected from the group consisting of chondroitinase ABC 1, chondroitinase ABC 11, chondroitinase AC, and chondroitinase B.

5. The chimeric protein of Claim 4 wherein the chondroitinase is chondroitinase ABC I.

6. The chimeric protein of Claim 2 wherein the matrix metalloproteinase is selected from the group consisiting of MMP-9, MMP-2, and pepsin.

7. The chimeric protein of Claim 1 wherein the second polypeptide possessing neurotrophic factor activity is selected from the group consisting of NGF, BDNF, NT-3, IGF, EGF, VEGF, FGF, PDGF, and TGF α and β.

8. The chimeric protein of any preceding claim wherein the first polypeptide is joined to the second polypeptide by a peptide linkage.

9. The chimeric protein of Claim 8 wherein the peptide linkage is an Fc portion of an immunoglobulin.

10. A pharmaceutical composition comprising a chimeric protein as claimed in any preceding claim in combination with a pharmaceutically acceptable carrier.

11. The chimeric protein of any one of Claims 1 to 9 for use in therapy.

12. Use of the chimeric protein of any one of Claims 1 to 9 for the manufacture of a medicament for enhancing nervous system repair and regeneration through administration of the chimeric protein to damaged cells of the nervous system.

## Patentansprüche

1. Ein chimäres Protein, das Folgendes beinhaltet:
1) ein erstes Polypeptid, das durch das Entfernen von Komponenten der extrazellulären Matrix, die das neuronale Wachstum und die neuronale Entwicklung hemmen, Matrixmodifizierungsaktivität besitzt und das aus Matrix abbauenden Enzymen ausgewählt ist; und
2) ein zweites Polypeptid, das neurotrophe Faktoraktivität für Nervenzellen besitzt,.
wobei das erste und das zweite Polypeptid in der Natur nicht gemeinsam auftreten und in dem chimären Protein miteinander verbunden sind.

2. Chimäres Protein gemäß Anspruch 1, wobei das erste Polypeptid aus der Gruppe, bestehend aus Chondroitinasen, Hyaluronidasen und Matrix-Metalloproteasen, ausgewählt ist.

3. Chimäres Protein gemäß Anspruch 2, wobei die Chondroitinase aus der Gruppe, bestehend aus Chondroitinase ABC Exolyase, Chondroitinase ABC Endolyase, Chondroitinase AC und Chondroitinase B, ausgewählt ist.

4. Chimäres Protein gemäß Anspruch 2, wobei die Chondroitinase aus der Gruppe, bestehend aus Chondroitinase ABC I, Chondroitinase ABC II, Chondroitinase AC und Chondroitinase B, ausgewählt ist.

5. Chimäres Protein gemäß Anspruch 4, wobei die Chondroitinase Chondroitinase ABC I ist.

6. Chimäres Protein gemäß Anspruch 2, wobei die Matrix-Metalloprotease aus der Gruppe, bestehend aus MMP-9, MMP-2 und Pepsin, ausgewählt ist.

7. Chimäres Protein gemäß Anspruch 1, wobei das zweite, neurotrophe Faktoraktivität besitzende Polypeptid aus der Gruppe, bestehend aus NGF, BDNF, NT-3, IGF, EGF, VEGF, FGF, PDGF und TGF α und β, ausgewählt ist.

8. Chimäres Protein gemäß einem der vorhergehenden Ansprüche, wobei das erste Polypeptid durch eine Peptidbindung mit dem zweiten Polypeptid verbunden ist.

9. Chimäres Protein gemäß Anspruch 8, wobei die Peptidbindung ein Fc-Teil eines Immunglobulins ist.

10. Eine pharmazeutische Zusammensetzung, die ein chimäres Protein gemäß einem der vorhergehenden Ansprüche in Kombination mit einem pharmazeutisch akzeptablen Träger beinhaltet.

11. Chimäres Protein gemäß einem der Ansprüche 1 bis 9 zur Verwendung bei einer Therapie.

12. Eine Verwendung des chimären Proteins gemäß einem der Ansprüche 1 bis 9 zur Herstellung eines Medikaments für die Verbesserung der Instandsetzung und Regeneration des Nervensystems durch die Verabreichung des chimären Proteins an beschädigte Zellen des Nervensystems.

## Revendications

1. Une protéine chimère comprenant :
1) un premier polypeptide possédant une activité de modification de matrice par retrait de composants de la matrice extracellulaire qui inhibent la croissance et le développement neuronaux et qui est sélectionné parmi des enzymes de digestion de matrice ; et
2) un deuxième polypeptide possédant une activité de facteur neurotrophique pour des cellules neurales,
lesdits premier et deuxième polypeptides n'étant pas présents ensemble dans la nature et étant joints l'un à l'autre dans ladite protéine chimère.

2. La protéine chimère de la revendication 1 où le premier polypeptide est sélectionné dans le groupe consistant en chondroïtinases, hyaluronidases, et métalloprotéinases matricielles.

3. La protéine chimère de la revendication 2 où la chondroïtinase est sélectionnée dans le groupe consistant en chondroïtinase ABC exolyase, chondroïtinase ABC endolyase, chondroïtinase AC, et chondroïtinase B.

4. La protéine chimère de la revendication 2 où la chondroïtinase est sélectionnée dans le groupe consistant en chondroïtinase ABC I, chondroïtinase ABC II, chondroïtinase AC, et chondroïtinase B.

5. La protéine chimère de la revendication 4 où la chondroïtinase est la chondroïtinase ABC I.

6. La protéine chimère de la revendication 2 où la métalloprotéinase matricielle est sélectionnée dans le groupe consistant en MMP-9, MMP-2, et pepsine.

7. La protéine chimère de la revendication 1 où le deuxième polypeptide possédant une activité de facteur neurotrophique est sélectionné dans le groupe consistant en NGF, BDNF, NT-3, IGF, EGF, VEGF, FGF, PDGF, et TGF α et β.

8. La protéine chimère de n'importe quelle revendication précédente où le premier polypeptide est joint au deuxième polypeptide par une liaison peptidique.

9. La protéine chimère de la revendication 8 où la liaison peptidique est une portion Fc d'une immunoglobuline.

10. Une composition pharmaceutique comprenant une protéine chimère telle que revendiquée dans n'importe quelle revendication précédente en combinaison avec un véhicule acceptable d'un point de vue pharmaceutique.

11. La protéine chimère de n'importe laquelle des revendications 1 à 9 destinée à être utilisée en thérapie.

12. Utilisation de la protéine chimère de n'importe laquelle des revendications 1 à 9 pour la fabrication d'un médicament destiné à renforcer la réparation et la régénération du système nerveux par le biais de l'administration de la protéine chimère à des cellules endommagées du système nerveux.
